# EUROPEAN PATENT SPECIFICATION

(11) **EP 3 512 517 B1**
(45) Date of publication and mention of the grant of the patent: **19.03.2025**
(21) Application number: 17851619.1
(22) Date of filing: 15.09.2017
(51) Int. Cl.: A61K 31/451, A61P 25/00, A61P 25/14, A61P 25/22, A61P 25/24

(54) **USE OF PRIDOPIDINE FOR THE TREATMENT OF ANXIETY AND DEPRESSION**
VERWENDUNG VON PRIDOPIDIN ZUR BEHANDLUNG VON ANGST UND DEPRESSION
UTILISATION DE PRIDOPIDINE POUR LE TRAITEMENT DE L'ANXIÉTÉ ET DE LA DÉPRESSION

(30) Priority: 15.09.2016 US 201662395259 P; 14.07.2017 US 201762532728 P
(43) Date of publication of application: 24.07.2019
(73) Proprietor: Prilenia Neurotherapeutics Ltd., Herzliya 4672561 (IL)
(72) Inventor: HAYDEN, Michael, 49131 Patach-Tikva (IL); POULADI, Mahmoud, Abdulhossein, Singapore 129789 (SG)
(74) Representative: Pearl Cohen Zedek Latzer Baratz UK LLP
(86) International application number: PCT/US2017/051814
(87) International publication number: WO 2018/053287

(56) References cited:
- WO-A1-2015/179522
- WO-A1-2016/138135
- US-A1- 2014 378 508
- US-A1- 2016 243 098
- SQUITIERI FERDINANDO ET AL: "One-year safety and tolerability profile of pridopidine in patients with Huntington disease", 27 February 2013 (2013-02-27), XP055978828, Retrieved from the Internet <URL:http://eolit-p.internal.epo.org/api/orders/1127126/pdf> [retrieved on 20221108]
- COMO PETER G. ET AL: "A controlled trial of fluoxetine in nondepressed patients with Huntington's disease", MOVEMENT DISORDERS, vol. 12, no. 3, 1 May 1997 (1997-05-01), US, pages 397 - 401, XP093214159, ISSN: 0885-3185, DOI: 10.1002/mds.870120319
- MUIJEN M. ET AL: "A comparative clinical trial of fluoxetine, mianserin and placebo in depressed outpatients", ACTA PSYCHIATRICA SCANDINAVICA., vol. 78, no. 3, 1 September 1988 (1988-09-01), DE, pages 384 - 390, XP093214162, ISSN: 0001-690X, DOI: 10.1111/j.1600-0447.1988.tb06353.x

## Description

### TECHNICAL FIELD.

The present invention relates to pharmaceutical compositions useful in reducing anxiety and/or depression in a subject.

### BACKGROUND

### Pridopidine

Pridopidine (4-[3-(methylsulfonyl)phenyl]-1-propyl-piperidine) (formerly known as ACR16) is a drug under development for treatment of Huntington's disease. The chemical name of pridopidine is 4-(3-(Methylsulfonyl)phenyl)-1-propylpiperidine and its Chemical Registry Number is CAS 346688-38-8 (CSID:7971505, 2016). The Chemical Registry number of pridopidine hydrochloride is 882737-42-0 (CSID:25948790 2016).

Pridopidine has been shown to modulate motor activity by either suppressing hyperactivity or enhancing hypoactivity. The neuroprotective properties of pridopidine are suggested to be attributed to its high affinity to the Sigma-1 receptor (S1R, binding IC50 ~ 100nM), while the motor activity of pridopidine may be mediated primarily by its low-affinity, antagonistic activity at the dopamine D2 receptor (D2R) (binding IC50 ~ 10µM) (Ponten 2010). Pridopidine shows low-affinity binding to additional receptors in the micromolar range.

The S1R is an endoplasmic reticulum (ER) chaperone protein which is implicated in cellular differentiation, neuroplasticity, neuroprotection and cognitive function in the brain. Recently, transcriptomic analysis of rat striatum showed that pridopidine treatment activates expression of the BDNF, dopamine receptor 1 (D1R), glucocorticoid receptor (GR), and the serine-threonine kinase protein kinase B (Akt)/phosphoinositide 3-kinase (PI3K) pathways, known to promote neuronal plasticity and survival and to be impaired in HD. Moreover, pridopidine gene expression profile showed a reversed pattern of the HD disease gene expression profile in a Q175 knock-in (Q175 KI) HD mouse model (Geva 2016). Pridopidine also enhances secretion of the neuroprotective brain-derived neurotrophic factor (BDNF) in a neuroblastoma cell line, in a S1R-dependent manner (Geva 2016). US 2014/378508 discloses a method of treating a human patient afflicted with Huntington's disease, comprising periodically orally administering to the patient a pharmaceutical composition comprising pridopidine or a pharmaceutically acceptable salt thereof such that greater than 90 mg of pridopidine is administered to the patient per day.

US 2016/243098 discloses a method of improving cognitive function in a subject comprising periodically administering pridopidine or a pharmaceutically acceptable salt thereof to the subject.

WO 2016/138135 discloses a method of treating a subject suffering from a neurodegenerative disease or neurodegenerative disorder comprising administering to the subject a therapeutically effective amount of a modulator of the Sigma-1 receptor.

Muijen et al. disclose a comparative clinical trial of fluoxetine, mianserin and placebo in depressed outpatients (Acta Psychiatr. Scand. 1988, 78, pages 384-390).

Como et al. disclose a controlled trial of fluoxetine in nondepressed patients with Huntington's Disease (Movement Disorders, Vol. 12, No. 3, 1997, pages 397-401).

### SUMMARY OF THE INVENTION

The present invention provides a pharmaceutical composition comprising pridopidine for use in reducing anxiety and/or depression in a subject in need thereof, wherein the anxiety or depression is not caused by Huntington's Disease.

In an embodiment, the anxiety is reduced by at least one scale unit of measure in the State-Trait Anxiety Inventory (STAI), the Fear Survey Schedule, Beck Anxiety Inventory (BAI), Brief Fear of Negative Evaluation Scale - BFNE, Clinician Administered PTSD Scale (CAPS), Daily Assessment of Symptoms - Anxiety, Generalized Anxiety Disorder 7 (GAD-7), Hamilton Anxiety Scale (HAM-A), Hospital Anxiety and Depression Scale (HADS-A), Leibowitz Social Anxiety Scale (LSAS), Overall Anxiety Severity and Impairment Scale (OASIS), Panic and Agoraphobia Scale (PAS), Panic Disorder Severity Scale (PDSS), PTSD Symptom Scale - Self-Report Version, Social Phobia Inventory (SPIN), Trauma Screening Questionnaire, Yale-Brown Obsessive Compulsive Scale (Y-BOCS), or the Zung Self-Rating Anxiety Scale.

In an embodiment, the depression is reduced by at least one scale unit of measure in the Hamilton Rating Scale for Depression (HAM-D), Beck Depression Inventory (BDI), Beck Hopelessness Scale, Centre for Epidemiological Studies - Depression Scale (CES-D), Patient Health Questionnaire, Center for Epidemiological Studies Depression Scale for Children (CES-DC), Clinically Useful Depression Outcome Scale, Diagnostic Inventory for Depression, Edinburgh Postnatal Depression Scale (EPDS), Inventory of Depressive Symptomatology, Geriatric Depression Scale (GDS), Hospital Anxiety and Depression Scale, Kutcher Adolescent Depression Scale (KADS), Major Depression Inventory (MDI), Montgomery-Åsberg Depression Rating Scale (MADRS), Mood and Feelings Questionnaire (MFQ), Zung Self-Rating Depression Scale, or the Cornell Scale for Depression in Dementia (CSDD).

In an embodiment, the subject is afflicted with an anxiety disorder which is generalized anxiety disorder (GAD), panic disorder, a phobic disorder, social phobia, agoraphobia, or a trauma- or stressor-related disorder.

In an embodiment, the subject is afflicted with a depressive disorder which is major depressive disorder, persistent depressive disorder, premenstrual dysphoric disorder, other depressive disorder, depressive disorder due to another medical condition, substance/medication-induced depressive disorder, perinatal depression, peripartum-onset depression, seasonal affective disorder, or psychotic depression.

In an embodiment, the subject has been diagnosed with anxiety only.

In an embodiment, the subject has been diagnosed with depression only.

In an embodiment, between 22.5 - 315 mg pridopidine is administered to the subject per day.

In an embodiment, the unit dose is administered once daily, more than once daily, or twice per day.

In an embodiment, the composition is administered orally.

In an embodiment, the pridopidine is in the form of pridopidine hydrochloride, hydrobromide, nitrate, perchlorate, phosphate, the sulphate, formate, acetate, aconate, ascorbate, benzenesulphonate, benzoate, cinnamate, citrate, embonate, enantate, fumarate, glutamate, glycolate, lactate, maleate, malonate, mandelate, methanesulphonate, naphthalene-2-sulphonate, phthalate, salicylate, sorbate, stearate, the succinate, tartrate, or toluene-p-sulphonate salt thereof.

### BRIEF DESCRIPTION OF THE FIGURES

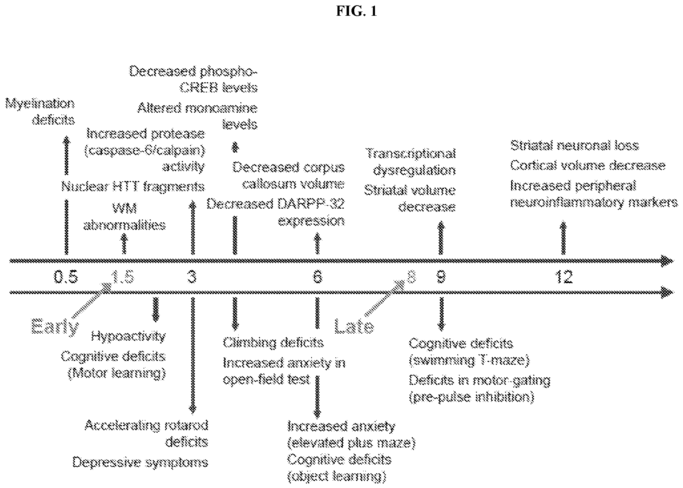

**Figure 1****.** (reference figure) **Pridopidine administration.** The timeline shows the early and late administration times of pridopidine relative to molecular, neuroanatomical and behavioural phenotypes in the rodent model of Huntington Disease, the YAC128 mice. The molecular and neuroanatomical phenotypes are listed along the upper horizontal line. The behavioural phenotypes are listed along the lower horizontal line. Age of the mice, in months, is presented between the upper and lower lines.

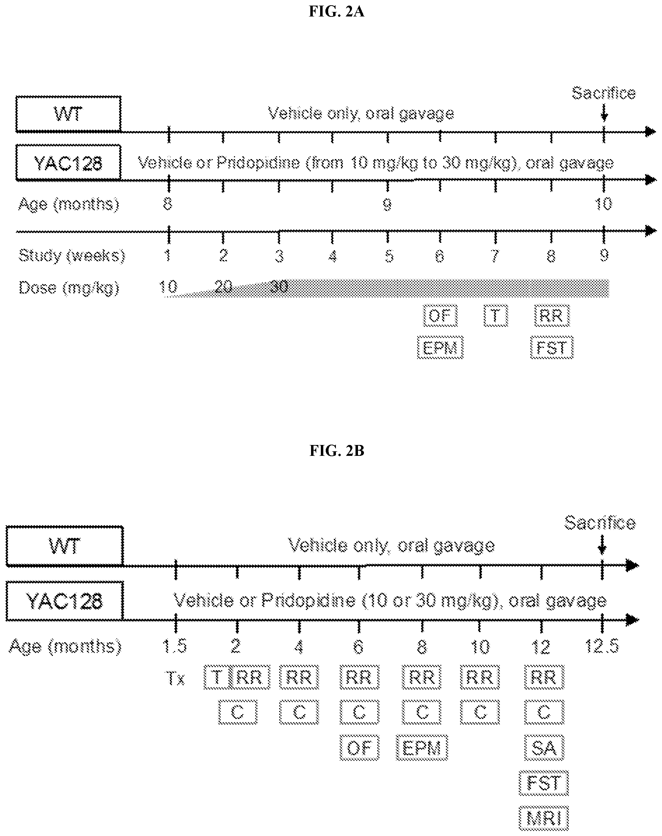

**Figures 2A and 2B****.** (reference figure) **Early and late treatment study designs.** (2A) Late pridopidine treatment study design. WT mice were administered vehicle (ddH2O) only, whereas YAC128 HD mice were administered either vehicle (ddH2O) or an escalating dose of pridopidine (10 mg/kg in week 1, 20 mg/kg in week 2, and 30 mg/kg in weeks 3-8). Treatment started at 8 months of age (manifest) and continued for 2 months. (2B) Early pridopidine treatment study design. WT mice were administered vehicle (ddH2O) only, whereas YAC128 HD mice were administered either vehicle (ddH2O) or pridopidine (10 or 30 mg/kg). Treatment started at 1.5 months of age (pre-manifest) and continued for 10.5 months. For both (2A) and (2B) a set of behavioural tests were carried out as indicated: T = motor learning, RR = accelerating rotarod, C = climbing test, OF = open field, EPM = elevated plus maze, SA = spontaneous activity test, FST = forced swim test; For (2B) magnetic resonance imaging (MRI) was also performed.

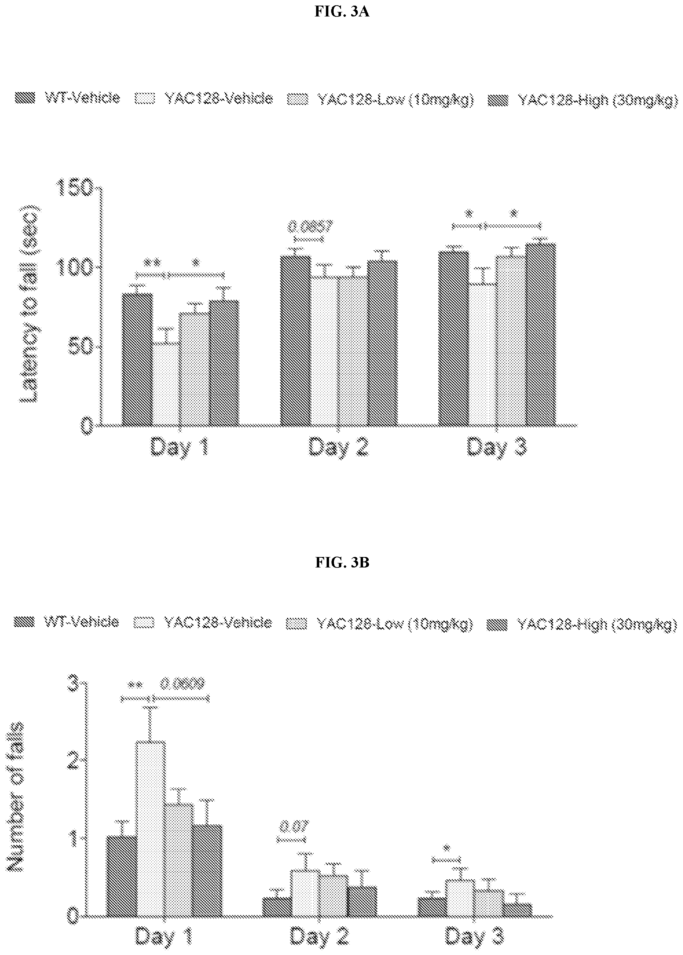

**Figures 3A** **(Latency to fall) and 3B (Number of falls).** (reference figures) **Cognitive function: motor learning.** YAC128 HD mice displayed motor learning deficits at 2 months compared to WT as signified by a shorter latency to fall (3A) and increased number of falls (3B). Vehicle-treated YAC128 HD mice displayed motor learning deficits in the rotarod training task at 2 months of age. High-dose pridopidine improved motor learning by increasing latency to fall in YAC128 HD mice (3A). Pridopidine treatment resulted in an improvement in motor learning in both WT and YAC 128 HD mice by reducing the number of falls (3B). Values shown as mean. Error bars represent SEM. Student's t-test (*p<0.05, **p<0.01); (N=20 per genotype/group).

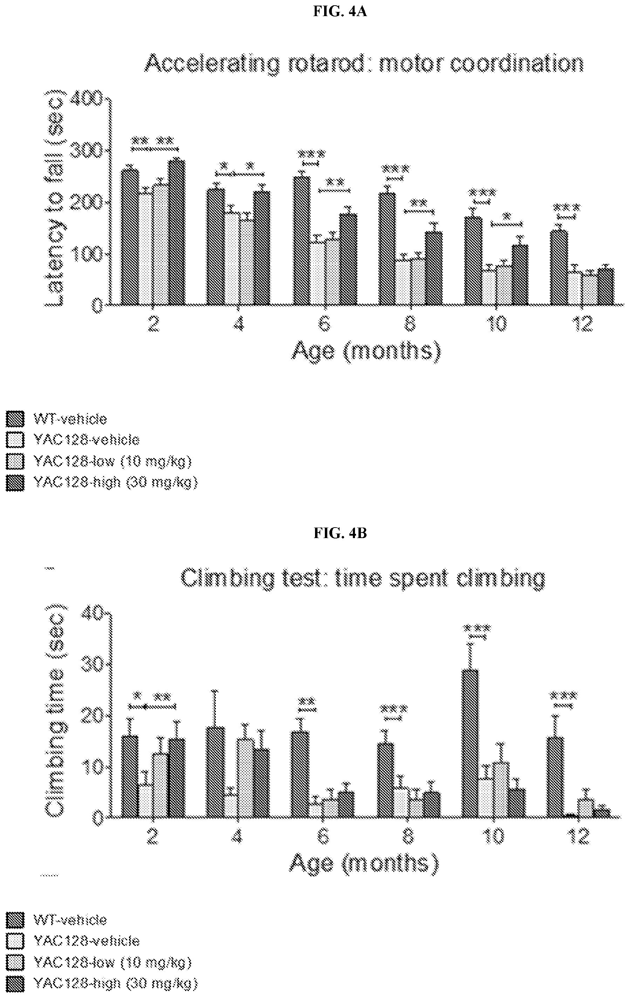

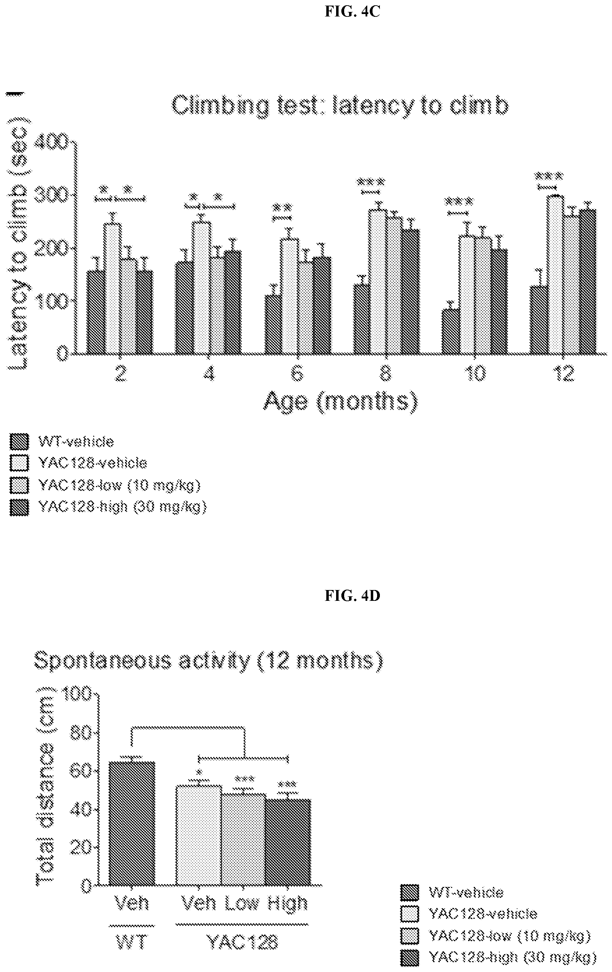

**Figures 4A-4D****:** (reference figures) **Early pridopidine treatment improves motor function in YAC128 HD mice.** Vehicle-treated YAC128 HD mice exhibited motor deficits in the accelerating rotarod. High-dose pridopidine improved motor performance as early as 2 months of age, maintained until 10 months. Low-dose pridopidine had no effect on rotarod performance (4A). Motor deficits in vehicle-treated YAC128 HD mice were also observed in the climbing test. High-dose pridopidine improved performance in climbing test by increasing climbing time at 2 months of age (4B) and decreasing latency to climb at 2 and 4 months of age (4C). Vehicle-treated YAC128 HD mice travelled a shorter distance in the spontaneous activity test. Pridopidine treatment (10 and 30 mg/kg) did not improve motor deficits in treated YAC128 HD mice (4D). In all graphs (4A-4D), Values shown as mean ± SEM; n = 11-21 WT-vehicle, n = 14-19 YAC128-vehicle, n = 18-20 YAC128-pridopidine (10 mg/kg), n = 16-20 YAC128-pridopidine (30 mg/kg); *p < 0.05, **p < 0.01, ***p < 0.001 by one-way ANOVA with Fisher's LSD post hoc analysis.

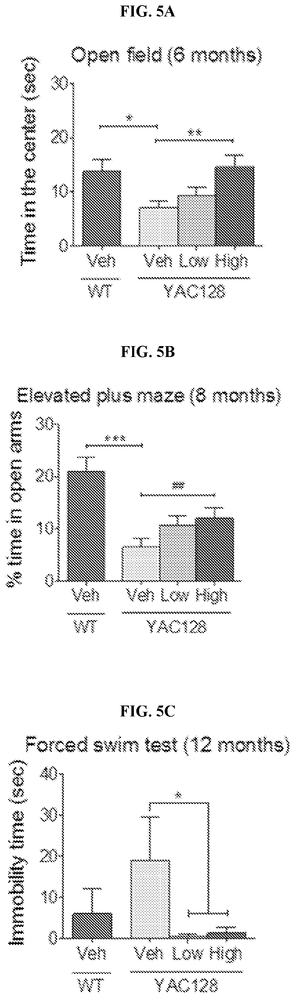

**Figures 5A-5C****.** (reference figures) **Pridopidine treatment improves Affective function.** **Figure 5A** **(Open field), 5B (Elevated plus maze) and 5C (Forced swim test).** YAC128 HD mice displayed increased anxiety-like behavior in the open field at 6 months (A) and elevated plus maze at 8 months of age (B) compared to WT mice. Early pridopidine treatment improves anxiety- and depressive-like phenotypes in YAC128 HD mice. Vehicle-treated YAC128 HD mice displayed anxiety-like phenotypes in the open field (5A) and elevated plus maze (5B). High-dose pridopidine increased the time spent in the center of the arena (5A) and in the open arms (5B), but no effect in mice receiving low-dose pridopidine was observed. Vehicle-treated YAC128 HD mice showed a trend towards an increased time spent immobile compared with vehicle-treated WT mice in the forced swim test. Both doses of pridopidine reduced depressive-like behavior (5C). Veh = Vehicle; Pri = Pridopidine; Low = 10 mg/kg; High = 30 mg/kg; M = males. (5A, 5B) Values shown as mean ± SEM; n = 16-17 WT-vehicle, n = 14-16 YAC128-vehicle, n = 19-20 YAC128-pridopidine (10 mg/kg), n = 16-17 YAC128-pridopidine (30 mg/kg); *p < 0.05, **p < 0.01, ***p < 0.001 by one-way ANOVA with Fisher's LSD post hoc analysis; ##p < 0.01 by paired Students t-test. (5C) Values shown as mean ± SEM; n = 4 (M) WT-vehicle, n = 8 (M) YAC128-vehicle, n = 9 (M) YAC128-pridopidine (10 mg/kg), n = 8 YAC128-pridopidine (30 mg/kg); *p < 0.05 by one-way ANOVA with Fisher's LSD post hoc analysis; ##p < 0.01 by paired Students t-test.

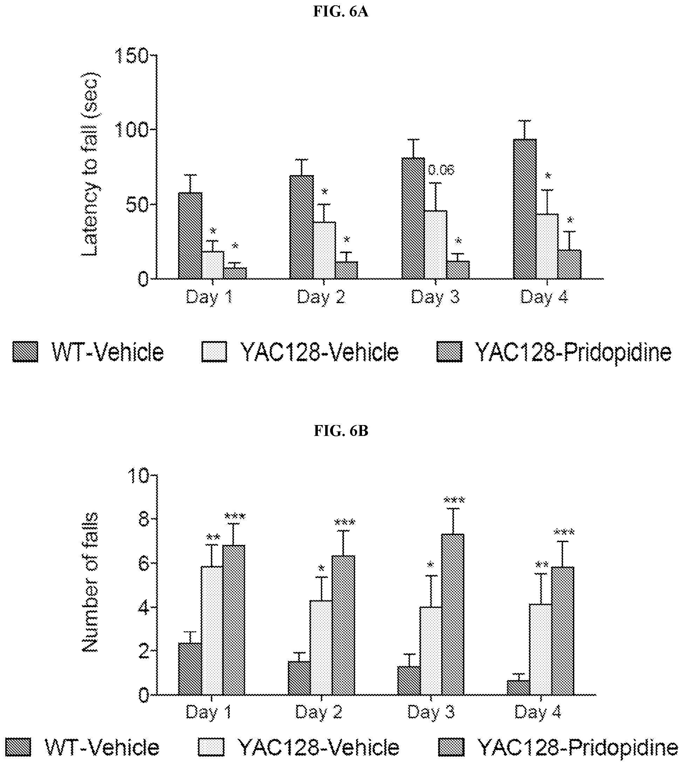

**Figure 6A** **(Latency to fall) and 6B (Number of falls).** (reference figures) **Cognitive function: motor learning in late stage pridopidine treated mice.** YAC128 HD mice displayed motor learning deficits at 9.5 months compared to WT as signified by a shorter latency to fall (6A) and increased number of falls (6B). Pridopidine treatment had no effect on motor learning performance at this age (6A, 6B). Values shown as mean. Error bars represent SEM. Student's t-test (*p<0.05, **p<0.01); (N=8-12 per genotype/group).

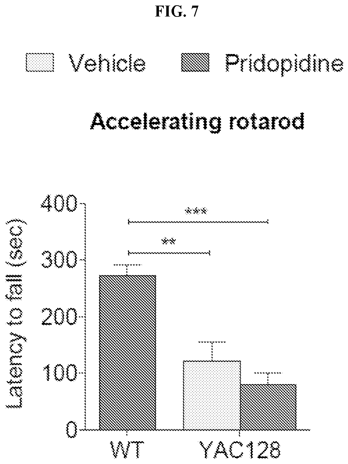

**Figure 7** (reference figure) **Motor function Accelerating rotarod in late stage pridopidine treated mice.** YAC128 HD mice displayed reduced motor performance at 10 months of age compared with WT mice as shown by a shorter latency to fall. Treatment with pridopidine did not improve motor performance of YAC128 HD mice at this age. Values shown as mean. Error bars represent SEM. Student's t-test ( **p<0.01, ****p<0.0001); (N=8-12 per genotype/group).

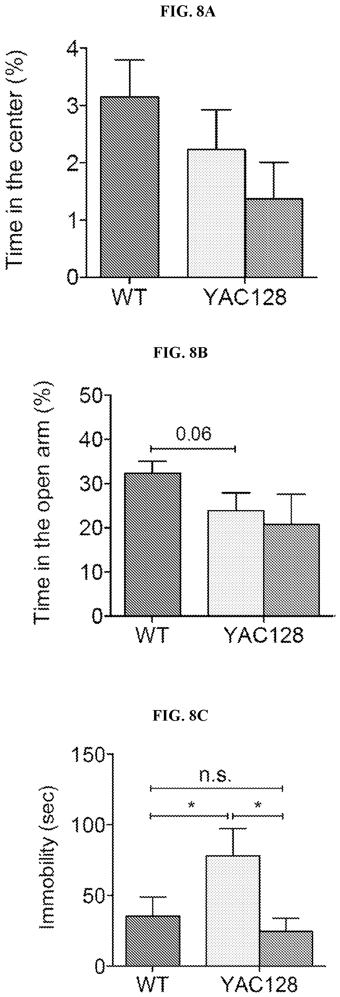

**Figure 8A** **(Open field), 8B (Elevated plus maze), and 8C.** (reference figures) **Affective function, Forced swim test in late stage pridopidine treated mice.** Treatment with pridopidine improved depressive-like phenotype of YAC128 HD mice in the forced swim test (8C). Values shown as mean. Error bars represent SEM. Student's t-test (*p<0.05, n.s. = not significant); (N=8-12 per genotype/group). The light grey bars represent vehicle treated; dark gray bars represent high dose (30 mg/kg).

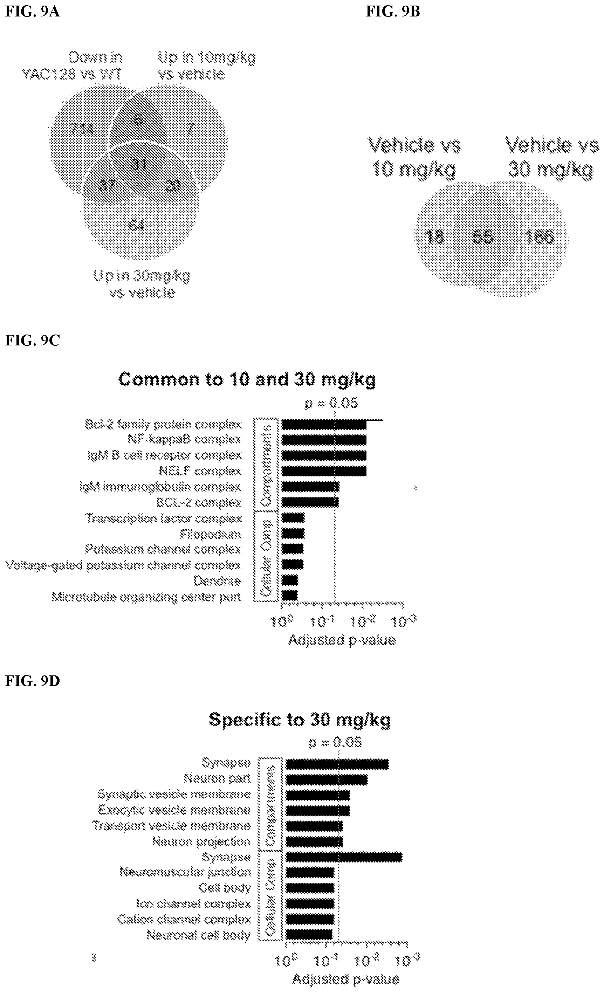

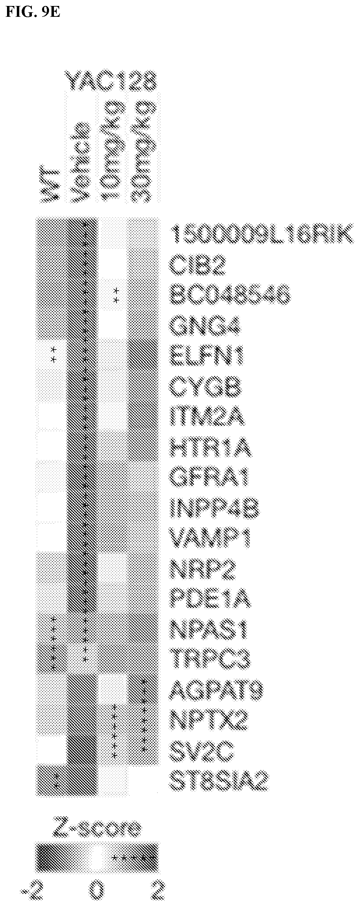

**Figure 9A****.** (reference figure) Pridopidine treatment reverses transcriptional deficits in the striatum of YAC128 HD mice. Overlap analysis of striatal genes downregulated in vehicle-treated YAC128 HD mice compared with vehicle-treated WT mice, and striatal genes upregulated in the striatum of pridopidine-treated YAC128 HD mice. **Figures 9B-9E****:** (reference figures) Functional annotation and cell type-specific analysis of DEGs in the striatum of YAC128 HD mice. (9B) Overlap in DEGs (relative to YAC128-vehicle) between the 10 mg/kg and 30 mg/kg pridopidine groups. (9C, 9D) Functional annotation of DEGs common to both 10 and 30 mg/kg (9C) or specific to 30 mg/kg (9D) enriched in COMPARTMENTS cellular component categories and gene ontology (GO) cellular component categories. (9E) Heatmap of gene transcripts enriched in cholinergic neurons specific to the 30 mg/kg pridopidine group. AGPAT9, NPTX2, SV2C and ST8SIA2 are upregulated.

### DETAILED DESCRIPTION OF THE INVENTION

The present invention provides a pharmaceutical composition comprising pridopidine for use in reducing anxiety and/or depression in a subject in need thereof, wherein the anxiety or depression is not caused by Huntington's Disease.

In an embodiment, anxiety is measured by the State-Trait Anxiety Inventory (STAI), the Fear Survey Schedule, Beck Anxiety Inventory (BAI), Brief Fear of Negative Evaluation Scale - BFNE, Clinician Administered PTSD Scale (CAPS), Daily Assessment of Symptoms - Anxiety, Generalized Anxiety Disorder 7 (GAD-7), Hamilton Anxiety Scale (HAM-A), Hospital Anxiety and Depression Scale (HADS-A), Leibowitz Social Anxiety Scale (LSAS), Overall Anxiety Severity and Impairment Scale (OASIS), Panic and Agoraphobia Scale (PAS), Panic Disorder Severity Scale (PDSS), PTSD Symptom Scale - Self-Report Version, Social Phobia Inventory (SPIN), Trauma Screening Questionnaire, Yale-Brown Obsessive Compulsive Scale (Y-BOCS), or the Zung Self-Rating Anxiety Scale.

In one embodiment, anxiety is reduced by at least one increment.

In an embodiment, depression is measured by Hamilton Rating Scale for Depression (HAM-D), Beck Depression Inventory (BDI), Beck Hopelessness Scale, Centre for Epidemiological Studies - Depression Scale (CES-D), Patient Health Questionnaire, Center for Epidemiological Studies Depression Scale for Children (CES-DC), Clinically Useful Depression Outcome Scale, Diagnostic Inventory for Depression, Edinburgh Postnatal Depression Scale (EPDS), Inventory of Depressive Symptomatology, Geriatric Depression Scale (GDS), Hospital Anxiety and Depression Scale, Kutcher Adolescent Depression Scale (KADS), Major Depression Inventory (MDI), Montgomery-Åsberg Depression Rating Scale (MADRS), Mood and Feelings Questionnaire (MFQ), Zung Self-Rating Depression Scale, or Cornell Scale for Depression in Dementia (CSDD).

In one embodiment, depression is reduced by at least one increment.

In another embodiment, the subject is afflicted with an anxiety disorder. In an embodiment, the anxiety disorder is generalized anxiety disorder (GAD), panic disorder, a phobic disorder, social phobia, agoraphobia, or trauma- and stressor-related disorders. In a further embodiment, the trauma- and stressor-related disorder is acute stress disorder (ASD), or posttraumatic stress disorder (PTSD).

In another embodiment, the subject is afflicted with a depressive disorder. In an embodiment, the depressive disorder is major depressive disorder, persistent depressive disorder, premenstrual dysphoric disorder, other depressive disorder, depressive disorder due to another medical condition, substance/medication-induced depressive disorder, perinatal depression, peripartum-onset depression, seasonal affective disorder, or psychotic depression.

In a further embodiment, the subject is not afflicted with Stage 1 or Stage 2 Huntington's disease. In one embodiment, the subject is not afflicted with Stage 1 Huntington's disease. In another embodiment, the subject is not afflicted with Stage 2 Huntington's disease.

In another embodiment, the subject is not afflicted with early stage Huntington's disease.

In an embodiment, the subject has greater than or equal to 36 CAG repeats in the huntingtin gene. In another embodiment, subject has greater than 44 CAG repeats in the huntingtin gene.

In some embodiments, the subject is presymptomatic. In other embodiments, the subject is symptomatic.

In one embodiment, the pharmaceutical composition is useful in reducing anxiety in a subject not afflicted with early stage Huntington's disease.

In another embodiment, the pharmaceutical composition is useful in reducing depression in a subject not afflicted with any one of the following: Stage 1 Huntington's disease, Stage 2 Huntington's disease, Stage 3 Huntington's disease, Stage 4 Huntington's disease and Stage 5 Huntington's disease.

In an embodiment, the subject is a human subject.

In an embodiment, the periodic administration is oral.

In an embodiment, between 22.5 - 315 mg pridopidine is administered to the subject per day. In another embodiment, 22.5 mg, 45 mg, 67.5, mg, 90 mg, 100 mg, 112.5 mg, 125 mg, 135 mg, 150 mg, 180 mg, 200 mg, 225mg, 250 mg, or 315 mg pridopidine is administered to the subject per day.

In an embodiment, the amount of pridopidine is administered by a unit dose of 22.5 mg, 45 mg, 67.5, mg, 90 mg, 100 mg, 112.5 mg, 125 mg, 135 mg, 150 mg, 180 mg, 200 mg, 225 mg, 250 mg, or 315 mg pridopidine.

In an embodiment, the unit dose is administered once daily.

In another embodiment, the pharmaceutical composition is administered for more than 26 weeks, at least 52 weeks, at least 54 weeks, at least 78 weeks, at least 104 weeks or more.

In an embodiment, the unit dose is administered more than once daily. In another embodiment, the unit dose is administered twice per day.

In an embodiment, the pridopidine is in the form of pridopidine hydrochloride.

In one embodiment, the anxiety and/or depression is reduced for at least 12 months.

In an embodiment, the subject has been diagnosed with anxiety only. In another embodiment, the subject is experiencing at least one symptom of anxiety, wherein the at least one symptom comprises restlessness, heart palpitations, hyperventilation, heavy sweating, muscle twitching, weakness, lethargy, insomnia, nausea, repetitive behavior, or any combination thereof.

In an embodiment, the subject has been diagnosed with depression only. In another embodiment the subject is experiencing at least one symptom of depression, and wherein the at least one symptom of depression comprises depressed mood, anhedonia, low energy levels, feelings of guilt, psychomotor retardation, agitation, suicidal ideations poor concentration and indecisiveness, or any combination thereof.

In an embodiment, a titration dose of an amount different from the intended dose is administered for a period of time at the start of the periodic administration. In some embodiments, the titration dose is half the amount of the intended dose. In another embodiment, the titration dose is administered in one administration per day and the intended dose is administered in two administrations per day. In one embodiment, the titration dose is administered for 7-21 or 7-14 days prior to the administration of the intended dose. In another embodiment, the titration dose is administered for 7 days, 14 days, or 21 days prior to the administration of the intended dose. The titration dose is preferably administered for fourteen days prior to the administration of the intended dose.

The pharmaceutical composition comprises an effective amount of pridopidine for reducing anxiety and/or depression in a subject.

The pharmaceutical composition may be provided as a package comprising:
a) the pharmaceutical composition comprising an amount of pridopidine for use according to the present invention; and
b) instructions for use of the pharmaceutical composition to reduce anxiety and/or depression in a subject.

The pharmaceutical composition may be provided in a therapeutic package for dispensing to, or for use in dispensing to, a subject, which comprises:
a) one or more unit doses, each such unit dose comprising an amount of pridopidine thereof, wherein the amount of said pridopidine in said unit dose is effective, upon administration to said subject, to reduce anxiety and/or depression in the subject, and
b) a finished pharmaceutical container therefor, said container containing said unit dose or unit doses, said container further containing or comprising labeling directing the use of said package in the reduction of anxiety and/or depression in said subject.

A method of predicting clinical responsiveness to pridopidine therapy in a subject afflicted with Huntington's disease, not forming part of the present invention, may comprise evaluating expression of a biomarker in the subject, so as to thereby predict clinical responsiveness to pridopidine, wherein the biomarker is a gene as described in this application. The method of predicting clinical responsiveness may further comprise predicting positive clinical responsiveness to pridopidine if the biomarker is upregulated in the subject. The method of predicting clinical responsiveness may further comprise predicting positive clinical responsiveness to pridopidine if the biomarker is suppressed in the subject. The subject may be identified as a pridopidine responder if expression of the biomarker is higher than a reference value. The subject may be identified as a pridopidine responder if expression level of the biomarker is lower than a reference value. If the subject is identified as a pridopidine responder, the subject may thereafter be administered a pharmaceutical composition comprising pridopidine.

### Terms

As used herein, and unless stated otherwise, each of the following terms shall have the definition set forth below.

As used herein, "administering to the subject" means the giving of, dispensing of, or application of medicines, drugs, or remedies to a subject to relieve, cure or reduce the symptoms associated with a disease, disorder or condition, e.g., a pathological condition. Oral administration is one way of administering the instant compounds to the subject.

As used herein, an "amount" or "dose" of pridopidine as measured in milligrams refers to the milligrams of pridopidine (4-[3-(methylsulfonyl)phenyl]-1-propyl-piperidine) present in a preparation, regardless of the form of the preparation. For example, a unit dose containing "90 mg pridopidine" means the amount of pridopidine base in a preparation is 90 mg, regardless of the form of the preparation. Thus, when in the form of a salt, e.g. pridopidine hydrochloride salt, the weight of the salt form necessary to provide a dose of 90 mg pridopidine would be greater than 90 mg due to the presence of the salt.

As used herein, a "unit dose", "unit doses" and "unit dosage form(s)" mean a single drug administration entity/entities.

As used herein, "about" in the context of a numerical value or range means ±10% of the numerical value or range recited or claimed.

As used herein, "effective" as in an amount effective to achieve an end means the quantity of a component that is sufficient to yield an indicated therapeutic response without undue adverse side effects (such as toxicity, irritation, or allergic response) commensurate with a reasonable benefit/risk ratio when used in the manner of this disclosure. For example, an amount effective to treat cognitive deficit. The specific effective amount varies with such factors as the particular condition being treated, the physical condition of the patient, the type of mammal being treated, the duration of the treatment, the nature of concurrent therapy (if any), and the specific formulations employed and the structure of the compounds or its derivatives.

As used herein, to "treat" or "treating" encompasses, e.g., inducing inhibition, regression, or stasis of a disorder and/or disease, e.g. depression, or alleviating, lessening, suppressing, inhibiting, reducing the severity of, eliminating or substantially eliminating, or ameliorating a symptom of the disease or disorder.

As used herein, "inhibition" of disease progression or disease complication in a subject means preventing, delaying or reducing the disease progression and/or disease complication in the subject. This includes, for example, delaying the progression of one of more symptoms in the subject, including delaying the progression of: cognitive impairment, intellectual disability, learning disabilities (e.g., having difficulty learning new skills), developmental delays (e.g., not sitting, walking, or talking at the same time as other children the same age), social and behavior problems (e.g., making eye contact, anxiety, trouble paying attention, hand flapping, acting and speaking without thinking, and being very active), anxiety and hyperactive behavior, hypersensitivity to sensory stimuli, altered gastrointestinal function, autistic symptoms (e.g., shyness, poor eye contact, and social anxiety in mild cases to hand flapping, hand biting and preservative speech in the severely affected), attention deficit and hyperactivity, behavioral disturbances (e.g., irritability, aggression and self-injurious behaviors), seizures, obsessive-compulsive behavior and altered gastrointestinal function.

In some embodiments, symptoms of anxiety include restlessness, heart palpitations, hyperventilation, heavy sweating, muscle twitching, weakness, lethargy, insomnia, nausea, repetitive behavior, or any combination thereof.

In some embodiments, symptoms of depression include depressed mood, anhedonia, low energy levels, feelings of guilt, psychomotor retardation, agitation, suicidal ideations poor concentration, indecisiveness, or any combination thereof.

A "pharmaceutically acceptable carrier" refers to a carrier or excipient that is suitable for use with humans and/or animals without undue adverse side effects (such as toxicity, irritation, and allergic response) commensurate with a reasonable benefit/risk ratio. It can be a pharmaceutically acceptable solvent, suspending agent or vehicle, for delivering the instant compounds to the subject.

As used herein, "pridopidine" means pridopidine base or a pharmaceutically acceptable salt thereof, as well as derivatives, for example deuterium-enriched version of pridopidine and salts. Examples of deuterium-enriched pridopidine and salts and their methods of preparation may be found in U.S. Patent Application Publication Nos. 2013-0197031, 2016-0166559 and 2016-0095847. In certain embodiments, pridopidine is a pharmaceutically acceptable salt, such as the HCl salt or tartrate salt.

Preferably, in any embodiments of the invention as described herein, the pridopidine is in the form of its hydrochloride salt.

"Deuterium-enriched" means that the abundance of deuterium at any relevant site of the compound is more than the abundance of deuterium naturally occurring at that site in an amount of the compound. The naturally occurring distribution of deuterium is about 0.0156%. Thus, in a "deuterium-enriched" compound, the abundance of deuterium at any of its relevant sites is more than 0.0156% and can range from more than 0.0156% to 100%. Deuterium-enriched compounds may be obtained by exchanging hydrogen with deuterium or synthesizing the compound with deuterium-enriched starting materials.

A dosage unit can be prepared for oral dosage forms, such as tablets, capsules, pills, powders, and granules. A dosage unit can be prepared for intravenous dosage forms.

### Pharmaceutically Acceptable Salts

The active compounds for use according to the invention may be provided in any form suitable for the intended administration. Suitable forms include pharmaceutically (i.e. physiologically) acceptable salts, and pre- or prodrug forms of the compound of the invention.

A "salt thereof" is a salt of the instant compound which has been modified by making acid or base salts of the compound. The term "pharmaceutically acceptable salt" in this respect, refers to the relatively non-toxic, inorganic and organic acid or base addition salts of compound of the present invention suitable for pharmaceutical use. Pharmaceutically acceptable salts may be formed by procedures well known and described in the art. One means of preparing such a salt is by treating a compound of the present invention with an inorganic base.

Examples of pharmaceutically acceptable salts include the non-toxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate, the phthalate, the salicylate, the sorbate, the stearate, the succinate, the tartrate, the toluene-p-sulphonate, and the like. Such salts may be formed by procedures well known and described in the art.

### Pharmaceutical Compositions

While the compounds for use according to the invention may be administered in the form of the raw compound, it is preferred to introduce the active ingredients, optionally in the form of physiologically acceptable salts, in a pharmaceutical composition together with one or more adjuvants, excipients, carriers, buffers, diluents, and/or other customary pharmaceutical auxiliaries.

In an embodiment, the invention provides pharmaceutical compositions comprising the active compounds or pharmaceutically acceptable salts thereof, together with one or more pharmaceutically acceptable carriers therefore, and, optionally, other therapeutic and/or prophylactic ingredients know and used in the art. The carrier(s) must be "acceptable" in the sense of being compatible with the other ingredients of the formulation and not harmful to the recipient thereof.

The pharmaceutical composition of the invention may be administered by any convenient route, which suits the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition of the invention can be manufactured by the skilled person by use of standard methods and conventional techniques appropriate to the desired formulation. When desired, compositions adapted to give sustained release of the active ingredient may be employed.

Further details on techniques for formulation and administration may be found in the latest edition of Remington's Pharmaceutical Sciences (Maack Publishing Co., Easton, PA).

It is understood that where a parameter range is provided, all integers within that range, and tenths thereof, are also provided by the invention. For example, "22 mg -300.0 mg" includes 22.0, 22.1 mg, 22.2 mg, 22.3 mg, 22.4 mg, etc. up to 300.0 mg.

### Anxiety Rating Scales

The anxiety rating scales listed herein are known to those skilled in the art. For example, the Beck Anxiety Inventory (BAI) is a measure of anxiety that has 21 items which are summed to obtain a total score from 0-63, in which a score of 0-9 is generally considered to mean normal or no anxiety; a score of 10-18 is generally considered to mean mild to moderate anxiety; a score of 19-29 is generally considered to mean moderate to severe anxiety; and a score of 30-63 is generally considered to mean severe anxiety (Julian 2011). Another anxiety rating scale is the Hospital Anxiety and Depression Scale-Anxiety (HADS-A) which has 7 items (Julian 2011). This scale evaluates common dimensions of anxiety and can be used to detect and quantify magnitude of symptoms of anxiety (Julian 2011). The total score for HADS-A can range from 0 to 21 and the following guidelines are recommended for the interpretation of scores: 0-7 for normal or no anxiety, 8-10 for mild anxiety, 11-14 for moderate anxiety, and 12-21 for severe anxiety (Julian 2011). Other anxiety rating scales are described in Spitzer 2006, Hamilton 1959, Leary 1983, Heimberg 1999, Norman 2006, Zigmond 1983, and Connor 2000. As used here, "reducing anxiety by at least one increment" means that the patient's anxiety as measured by at least one of the specific anxiety rating scales is lessened. For example, the STAI is an anxiety rating scale which has two subtest, a State Anxiety Scale (S-Anxiety) and a Trait Anxiety Scale (T-Anxiety) (Julian 2011). The range of scores for each subtest is 20-80 with a higher score indicating greater anxiety (Julian 2011). Therefore, a subject obtains a score of between 40 and 160 after completing the STAI. The subject's anxiety is reduced by at least one increment if the subject's STAI score is reduced by 1 or more points.

The patient anxiety may also be measured by one of the following anxiety rating scales: State-Trait Anxiety Inventory (STAI), the Fear Survey Schedule, Beck Anxiety Inventory (BAI), Brief Fear of Negative Evaluation Scale - BFNE, Clinician Administered PTSD Scale (CAPS), Daily Assessment of Symptoms - Anxiety, Generalized Anxiety Disorder 7 (GAD-7), Hamilton Anxiety Scale (HAM-A), Hospital Anxiety and Depression Scale (HADS-A), Leibowitz Social Anxiety Scale (LSAS), Overall Anxiety Severity and Impairment Scale (OASIS), Panic and Agoraphobia Scale (PAS), Panic Disorder Severity Scale (PDSS), PTSD Symptom Scale - Self-Report Version, Social Phobia Inventory (SPIN), Trauma Screening Questionnaire, Yale-Brown Obsessive Compulsive Scale (Y-BOCS), and the Zung Self-Rating Anxiety Scale.

### Depression Rating Scales

The depression rating scales listed herein are known to those skilled in the art. For example, Hamilton Depression Rating Scale (HAM-D) may be used to determine a patient's level of depression. The HAM-D lists 21 items, but scoring is based on the first 17 in which 0-6 is considered normal, 7-17 is considered mild depression, 18-24 is considered moderate depression, and 25 and greater is considered severe depression (Cusin 2009). Other depression rating scales are described in Bech 2001, Bech 2006, Strik 2001, and Cusin 2009.

As used here, "reducing depression by at least one increment" means that the patient's depression as measured by at least one of the specific depression rating scales is lessened. For example, in the HAM-D scale discussed above, the subject's depression is reduced by at least one increment if the subject's HAM-D score is reduced by 1 or more points.

The patient's depression may also be measured by one of the following depression rating scales: Hamilton Rating Scale for Depression (HAM-D), Beck Depression Inventory (BDI), Beck Hopelessness Scale, Centre for Epidemiological Studies - Depression Scale (CES-D), Patient Health Questionnaire, Center for Epidemiological Studies Depression Scale for Children (CES-DC), Clinically Useful Depression Outcome Scale, Diagnostic Inventory for Depression, Edinburgh Postnatal Depression Scale (EPDS), Inventory of Depressive Symptomatology, Geriatric Depression Scale (GDS), Hospital Anxiety and Depression Scale, Kutcher Adolescent Depression Scale (KADS), Major Depression Inventory (MDI), Montgomery-Åsberg Depression Rating Scale (MADRS), Mood and Feelings Questionnaire (MFQ), Zung Self-Rating Depression Scale, or Cornell Scale for Depression in Dementia (CSDD).

### Stages of Huntington's Disease

Many clinicians and diagnosticians adopt the Shoulson and Fahn rating scale, based on Total Functional Capacity (TFC) scores, to follow progression of HD. This rating scale groups total TFC scores into five stages of disease, with lower stages indicating more intact functioning. Table 1, below, provides the TFC scores, average years from diagnosis and broad guidelines for typical care level for each stage of disease (Johnson 2014).

**Table 1 HD stages corresponding to TFC scores and care level**

| Stage | TFC score | Years since motor diagnosis | Typical abilities and care level |
|---|---|---|---|
| 1 | 11-13 | 0-8 | Able to work at least part time, may require slight assistance in one of finances, domestic chores or ADL basic functions. |
| 2 | 7-10 | 3-13 | Unable to work, requires some assistance in some basic functions. |
| 3 | 3-6 | 5-16 | Unable to work, requires major assistance in most basic functions. |
| 4 | 1-2 | 9-21 | Requires major assistance in all basic functions and although comprehension may be intact requires assistance to act. |
| 5 | 0 | 11-26 | Requires major assistance in all basic functions and full time nursing care. |

This invention will be better understood by reference to the Experimental Details which follow, but those skilled in the art will readily appreciate that the specific experiments detailed are only illustrative of the invention as described more fully in the claims which follow thereafter.

### EXPERIMENTAL DETAILS

Reference Example 1: Evaluation of pridopidine in transgenic YAC128 mouse model of Huntington Disease.

Pridopidine is currently in clinical development for Huntington disease (HD) and investigations to increase the understanding of its therapeutic benefit and mode of action are ongoing.

This study investigated the efficacy and mechanism of action of pridopidine using the transgenic YAC128 mouse model of HD (Garcia-Miralles 2016). Pridopidine was administered to animals starting at early (1.5 months of age) or late stages of disease (8 months of age). In the early treatment cohort, animals were divided into three groups receiving 0, 10, or 30 mg/kg of pridopidine for a period of 10.5 months. In the late cohort, animals were divided into two groups receiving either 0 mg/kg or an escalating dose of pridopidine (10 mg/kg in week 1, 20 mg/kg in week 2, and 30 mg/kg in weeks 3-8). Pridopidine treated animals were evaluated using a battery of behavioral tests. Analysis reveals that chronic treatment with pridopidine improves motor coordination, anxiety-like and depressive-like phenotypes in the YAC128 HD mice.

### Materials and Methods:

Animals. Male and female YAC128 HD mice (line 53) expressing a full-length human HTT (huntingtin) transgene with 128 CAG repeats, maintained on the FVB/N strain were used. Mice were bred at the Biological Resource Centre (Agency for Science, Technology and Research, ASTAR), and group-housed with littermates of mixed genotype. Animals were maintained under a 12-h light cycle (lights on at 09:00) in a clean facility, and given free access to food and water. Experiments were performed with the approval of the Institutional Animal Care and Use Committee at the Biomedical Sciences Institute (ASTAR) and in accordance with their approved guidelines.

Administration of pridopidine. Pridopidine was dissolved in sterile water. Pridopidine and vehicle were administered daily by oral gavage for five days/week for 10.5 months for the early treatment cohort and 8 weeks for the late treatment cohort. Mice received vehicle (sterile water), 10 mg/kg of pridopidine, or 30 mg/kg of pridopidine at a volume of 4 mL/kg. Animals were weighed every two weeks to ensure the correct dose was maintained.

### Study design.

Late treatment cohort: Pridopidine was administered to animals in advanced stages of disease (8 months of age). At this age, mice present striatal atrophy and profound behavioural deficits. Animals were divided into two groups receiving either 0 mg/kg or an escalating dose of pridopidine (10 mg/kg in week 1, 20 mg/kg in week 2, and 30 mg/kg in weeks 3-8). A battery of behavioural tests was performed between 9.5 and 10 months of age. Mice were tested on motor learning (rotarod training), motor function (rotarod test), and psychiatric function (open field, elevated plus maze, and forced swim test) and were sacrificed following completion of behavioural testing at 10 months of age.

Early treatment cohorts: Pridopidine was administered to animals (two cohorts) in the early stages of disease (1.5 months of age). Mice were divided into four groups. Two groups of YAC128 HD mice received pridopidine at a dose of 10 or 30 mg/kg, whereas the remaining groups, WT mice and YAC128 HD mice, received an equivalent volume of vehicle. One cohort was behaviourally tested every two months commencing at 2 months of age. Mice were tested on motor learning (rotarod training), motor function (rotarod and climbing tests), and psychiatric function (open field, elevated plus maze, and forced swim test). Tests were conducted at a set time during the day, prior to drug administration. Subsequently, these mice were scanned by magnetic resonance imaging (MRI) and sacrificed at 12.5 months of age. The other cohort was scanned by MRI at 7 and 10 months of age and was used for pharmacokinetic (PK) measurement and RNA-Seq analysis at 11 months of age.

Pridopidine was administered to animals starting at early (1.5 months of age) or late stages of disease (8 months of age) 5 days a week by oral gavage. Table 2 shows the treatment protocol for early stage disease cohort (1.5 mo old YAC128 HD mice). Table 3 shows the treatment protocol for late stage disease cohorts (8 mo old YAC128 HD mice).

**Table 2. Early stage pridopidine treatment**

| Pridopidine early treatment groups | | | | |
|---|---|---|---|---|
| Genotype | Pridopidine dose (mg/kg) | Sex | Number | Total N/treatment |
| WT | 0 | F | 10 | 20 |
| | | M | 10 | |
| YAC128 | 0 | F | 10 | 20 |
| | | M | 10 | |
| YAC128 | 10 | F | 10 | 20 |
| | | M | 10 | |
| YAC128 | 30 | F | 10 | 20 |
| | | M | 10 | |

**Table 3. Late stage pridopidine treatment**

| Pridopidine late treatment groups | | | | | | |
|---|---|---|---|---|---|---|
| Genotype | Pridopidine dose (mg/kg) | | | Sex | Number | Total N/treatment |
| WT | 0 | 0 | 0 | F | 3 | 12 |
| | | | | M | 9 | |
| YAC128 | 0 | 0 | 0 | F | 3 | 9 |
| | | | | M | 9 | |
| YAC128 | 10 | 20 | 30 | F | 3 | 10 |
| | | | | M | 7 | |

### Test of cognitive function

*Rotarod test of motor learning.* The rotarod training is used as a measure to assess cognitive function in rodents. For training, mice were given three trials (120 sec each) per day spaced 1 hour apart at a fixed speed of 18 rpm for three or four consecutive days.

### Tests of motor function

Accelerating rotarod test. The rotarod test is designed to evaluate motor coordination and balance in rodents. The test consists of three trials spaced 2 hour apart where the rotarod accelerated from 5 to 40 rpm over 5 min. Rotarod scores are the average of three trials. The maximum score is 300 sec.

Climbing test. The climbing test is used to assess motor function in rodents. Mice were placed at the bottom end of a closed-top wire mesh cylinder and their behavior was monitored for 5 min. The sum of climbing time for the 5 min trial is the total time spent climbing for each mouse.

### Tests of affective function

*Anxiety-like behavioral tests.* The open-field (OF) and elevated plus maze (EPM) tests are used to assess anxiety-like behavioral in rodents. The time spent in the center of the arena in the OF and the time spent in the open arms of the maze in the EPM are considered measures of anxiety-like behavior.

*Depressive-like behavioral tests.* The Porsolt forced-swim test is used to assess depressive-like behavior in rodents. The time spent immobile is considered a measure of depressive-like behavior. Immobility scores for each mouse were determined by manual scoring.

### Results

Detailed results are shown in the figures and described in the description of the figures. Results of early stage treatment are shown in Figures 3-5. Results of late stage treatment are shown in Figures 7-9. These results demonstrate that early treatment with pridopidine improved motor function in YAC128 HD mice. However, late treatment with pridopidine was not as effective to improve motor function in YAC128 HD mice. Furthermore, anxiety-like behavior of YAC128 HD mice was improved in the early treatment cohort, whereas depressive-like behavior was improved in both early and late treatment cohort. Altogether, these results show that pridopidine improves motor and affective symptoms in the YAC128 HD animal model.

These results also show that early treatment with pridopidine results in improvements in motor learning and coordination, and in psychiatric-like features in the YAC128 mouse model of HD. The effects of treatment showed dose dependence, with 30 mg/kg resulting in greater overall benefit compared with the 10 mg/kg dose. The improvements were seen throughout the disease course, with improved motor performance lasting from 2 to 10 months of age, reduced anxiety-like phenotypes at 6 and 8 months of age, and amelioration of depressive-like behaviour at 12 months of age.

The improvements in behavioural outcomes are not likely to represent acute effects given pridopidine's short half-life and the fact that on test days, pridopidine was administered after the behavioural assays were completed. Furthermore, the effects appear to be HD-specific as no effects were seen in treated WT mice (data not shown). In contrast, when pridopidine was administered to mice at a later time point when the disease was clearly manifest, the functional benefits were limited, although improvements in depressive-like behaviour were noted.

These findings show that early administration of pridopidine exhibits anxiolytic (30 mg/kg) and antidepressant (10 and 30 mg/kg) properties in YAC128 HD mice, and therefore may be used for the treatment of HD-associated psychiatric symptoms. This data also shows that late administration of pridopidine in manifest YAC128 HD mice is efficacious to treat depressive-like phenotypes at late stages of HD.

### Reference Example 2: Effect of early pridopidine treatment on HD-related neuropathology in YAC128 HD mice.

Given that improvements in behavioural outcomes were mainly observed in animals that received early pridopidine treatment, pridopidine's efficacy on HD-related neuropathology in this particular cohort was evaluated. Striatal atrophy and white matter abnormalities are neuropathological features observed in patients with HD that have also been described in YAC128 HD mice (Teo 2016, Garcia-Miralles 2016, Carroll 2011). To assess whether pridopidine could also have therapeutic effects on HD-related neuropathology, striatal and corpus callosum (CC) volume was measured by structural magnetic resonance imaging. At 12 months of age, vehicle-treated YAC128 HD mice displayed decreased striatal and CC volume compared with vehicle-treated WT mice (one-way ANOVA with Fisher's LSD post hoc analysis; p < 0.001 for striatum and CC). However, no effect of pridopidine treatment was observed in these two measures in treated YAC128 HD mice (one-way ANOVA with Fisher's LSD post hoc analysis; p > 0.05 for both doses).

Following structural MRI analysis, mice were perfused and brains were examined by histological and stereological methods. Consistent with earlier reports (Slow 2003), vehicle-treated YAC128 HD mice exhibited reduced forebrain weight compared with vehicle-treated WT mice (one-way ANOVA with Fisher's LSD *post hoc* analysis; p < 0.001). Pridopidine treatment had no effect on forebrain weight in treated YAC128 HD mice (one-way ANOVA with Fisher's LSD *post hoc* analysis; p > 0.05 for both doses of pridopidine compared with vehicle-treated YAC129 HD mice). Subsequently, brains were coronally sectioned and stained with NeuN, a neuronal nuclear marker, to determine striatal volume and striatal neuronal loss by stereological assessment. Consistent with the results we obtained by structural MRI, vehicle-treated YAC128 HD mice displayed decreased striatal volume compared with vehicle-treated WT mice (one-way ANOVA with Fisher's LSD *post hoc* analysis; p < 0.001); however, this phenotype was not rescued with pridopidine treatment (one-way ANOVA with Fisher's LSD *post hoc* analysis; p > 0.05 for both doses of pridopidine compared with vehicle-treated YAC128 HD mice). Because striatal atrophy can be accompanied by striatal neuronal loss (Slow 2003), it was estimated by the total number of striatal neurons. Stereological counts of NeuN-positive neurons in the striatum revealed no significant differences between vehicle-treated YAC128 HD and WT mice (one-way ANOVA with Fisher's LSD *post hoc* analysis; p > 0.05) as well as between pridopidine- and vehicle-treated YAC128 HD mice (one-way ANOVA with Fisher's LSD *post hoc* analysis; p > 0.05 for both doses of pridopidine). DARPP32 expression was further assessed in medium-sized spiny neurons by quantification of DARPP32 immunoreactivity at 12 months of age. Although reduced expression of DARPP32 has been described in YAC128 HD mice (Southwell 2015), only a slight reduction in optical density (OD) in vehicle-treated YAC128 HD mice was detected compared with vehicle-treated WT mice, which did not reach statistical significance (one-way ANOVA with Fisher's LSD *post hoc* analysis; p = 0.113). Treatment with the high dose of pridopidine resulted in a slight increment in OD in treated YAC128 HD mice, although this difference did not reach statistical significance (one-way ANOVA with Fisher's LSD *post hoc* analysis; p = 0.079 compared with vehicle-treated YAC128 HD mice). No effect of the low dose of pridopidine was observed (one-way ANOVA with Fisher's LSD *post hoc* analysis; p = 0.147 compared with vehicle-treated YAC128 HD mice). These results indicate that early administration of pridopidine starting before manifestation of disease phenotypes in YAC128 HD mice at the doses used in this study (10 and 30 mg/kg) is not sufficient to rescue HD-related neuropathology in YAC128 HD mice.

### Reference Example 3: Early pridopidine treatment reverses transcriptional deficits in YAC128 HD mice.

To better understand the mechanism of action of early pridopidine treatment, RNA sequencing (RNA-seq) analysis was performed to investigate the transcriptional profile of the mouse striatum at 11 months of age.

Since transcriptional dysregulation is a well characterized feature of HD (Kuhn 2007), RNA-seq data between vehicle-treated WT and YAC128 HD mice was first compared and 1346 differentially expressed genes (DEG) were found, for which expression was significantly changed in the striatum between both genotypes (Heatmap; p<0.05). Out of these DEGs, 788 genes were downregulated and 558 genes were upregulated under disease conditions.

Given that pridopidine treatment improved HD symptoms but failed to rescue striatal or corpus callosal atrophy, it was considered whether early pridopidine treatment could influence the health of striatal neurons. The effect of pridopidine treatment on five striatally-enriched genes known to be specifically downregulated with disease progression in HD was investigated. These genes were protein phosphatase 1 regulatory inhibitor subunit 1B (*Ppp1r1b* or Darpp32), cannabinoid receptor 1 (*Cnr1*), dopamine receptors D1 and D2 (*Drd1* and *Drd2,* respectively), and proenkephalin (*Penk*) (Pouladi 2012). As expected, all five genes showed a reduction in the number of reads in vehicle-treated YAC128 HD mice compared with vehicle-treated WT mice (limma; p < 0.001 for all five genes). Interestingly, dose- and gene-specific effects of pridopidine treatment were observed on the transcriptome profile of striatal-enriched genes. Both doses of pridopidine reversed *Drd1* and *Drd2* deficits in treated YAC128 HD mice (limma; p < 0.01 for low dose of pridopidine, and p < 0.001 for high dose of pridopidine in D1R; p < 0.05 for both doses of pridopidine in D2R). *Ppp1r1b* (Darpp32) and *Penk* reads were also increased in the striatum of treated YAC128 HD mice after treatment with the lower (10 mg/kg) and higher (30 mg/kg) dose of pridopidine, respectively (limma; p < 0.05 for Ppp1r1b and p < 0.05 for Penk compared with vehicle-treated YAC128 HD mice). However, pridopidine was not able to reverse Cnr1 deficits in treated YAC128 HD mice at the doses used in this study (limma; p > 0.05 for both doses of pridopidine compared with vehicle-treated YAC128 HD mice).

To gain further insight into the mechanism of pridopidine's action, an unbiased genome-wide transcriptional analysis was performed for genes that were up- or downregulated in YAC128 HD mice and that were reversely regulated by pridopidine treatment. Overlap analysis revealed 74 significant DEGs, for which the number of reads was downregulated in vehicle-treated YAC128 HD mice and upregulated following pridopidine treatment (10 mg/kg and/or 30 mg/kg). Of those, 31 genes were common to all three groups. When a search was done for genes that were upregulated in vehicle-treated YAC128 HD mice and downregulated following pridopidine treatment, overlap analysis identified 10 genes that were downregulated by pridopidine which were not shared between both doses of pridopidine.

Close examination of the list of 74 genes that were transcriptionally reversed by pridopidine treatment identified a number of genes previously implicated in HD including *Gpx6, Kdm3a, Rheb* and *Dusp1.* These genes were downregulated in vehicle-treated YAC128 HD mice (limma; p < 0.001 for *Gpx6, Kdm3a* and *Rheb,* p < 0.01 for *Dusp1*) and this reduction was reversed by both doses of pridopidine as signified by a larger number of reads in pridopidine-treated YAC128 HD mice (limma; p < 0.01 for *Gpx6, Kdm3a, Rheb* and p < 0.001 for *Dusp1* for low dose; p < 0.001 for *Gpx6, Kdm3a, Rheb* and *Dusp1* for high dose). Altogether, these results from RNA-seq analysis revealed that pridopidine treatment reverses striatal transcriptional deficits of genes implicated in HD, showing a beneficial effect of pridopidine at a molecular level.

The biological basis for the greater behavioural improvements seen in the high dose (30 mg/kg) group relative to the low dose (10 mg/kg) group was explored by comparing the DEGs seen in each pridopidine group relative to the vehicle-treated YAC128 HD control group. Functional annotation of DEGs common to both the 10 mg/kg and 30 mg/kg groups using Enrichr (Kuleshov 2016) revealed 9 significantly enriched COMPARTMENTS (Desplats 2007) cellular component categories, including Bcl-2 (adjusted p = 0.003) and NF-kB (adjusted p = 0.036) complex members, and no enriched gene ontology (GO) cellular component categories. In contrast, DEGs specific to the 30 mg/kg group were enriched for 7 COMPARTMENTS cellular component categories such as Synapse (adjusted p = 0.003), Synaptic vesicle membrane (adjusted p = 0.025) and Neuron Projection (adjusted p = 0.037), as well as the GO cellular component synapse category (adjusted p = 0.001).

To further investigate pridopidine dose effects, DEGs using cell type-specific expression analysis were examined (Fenster 2011). Whereas DEGs common to both the 10 mg/kg and 30 mg/kg groups were enriched in genes expressed in striatal medium spiny and cortical project neurons, DEGs specific to the 30 mg/kg group were additionally enriched in transcripts expressed in cholinergic neurons. Inspection of the 19 DEGs annotated as being enriched in cholinergic neurons reveals that while their pattern of expression is similar between the 10 mg/kg and 30 mg/kg groups, the magnitude of change in their level of expression (relative to the YAC128 vehicle group) is greater in the 30 mg/kg compared to the 10 mg/kg group. Furthermore, a number of these genes have been previously implicated in HD such as Cib2(Czeredys 2013), Npas1 (van Roon-Mom 2008), St8sia2 (Desplats 2007), and Gng4 (Fenster 2011). These results show that the dosage effects seen in the behavioural assays are associated with differential transcriptional patterns, and further implicate synaptic changes and the cholinergic system in the greater functional improvements seen with the 30 mg/kg pridopidine dose.

### Discussion

Support for pridopidine's protective molecular effects is provided by the reversal of a substantial proportion of striatal transcriptional deficits in the pridopidine-treated YAC128 HD mice. Transcriptional dysregulation is a well-documented pathological feature of HD (Kuhn 2007). The analysis presented herein revealed that pridopidine treatment rescued transcriptional deficits in key pathways previously implicated in HD. For example, Gpx6 and Kdm3a were previously linked to HD in an unbiased synthetic lethal screen in the R6/2 mouse model of HD (Shema 2014). *Gpx6* (glutathione peroxidase 6) belongs to the glutathione peroxidase family and functions in the detoxification of hydrogen peroxidase. Knockdown of Gpx6 was shown to lead to loss of striatal neurons expressing mHTT, whereas overexpression of Gpx6 resulted in neuronal rescue, as measured by DARPP-32 expression, and improved motor function (Shema 2014). Similarly, deficits in the levels of the mTORC1 regulator Rheb in YAC128 HD mice were reversed by pridopidine treatment. Increasing Rheb activity was recently shown to ameliorate a number of cellular and molecular deficits in HD mice, including mitochondrial deficits and cholesterol dyshomeostasis. Finally, pridopidine treatment rescued deficits in DUSP1, a member of the mitogen-activated protein kinase family of phosphatases recently shown to be protective in animal models of HD. Collectively, these findings show that the transcriptional deficits reversed by pridopidine treatment contributes to its beneficial effects in YAC128 HD mice.

Support for synaptoprotective activity of pridopidine is also provided by the analysis of DEGs specific to the 30 mg/kg pridopidine group which showed greater improvement in behavioural assays. Functional annotation of the DEGs revealed enrichment for synapse and synaptic vesicle-associated genes. In addition, cell type-specific expression analysis showed that DEGs specific to the 30 mg/kg pridopidine dose were enriched in genes expressed in cholinergic neurons, showing a possible role for these neurons in mediating pridopidine's beneficial effects. Dysfunction of the cholinergic system has been reported to play a role in the pathophysiology of several movement disorders, including Parkinson's disease (PD) and HD (van Waarde 2011, Benarroch 2012, Pisani 2007). Importantly, S1R (sigma 1 receptor) agonists have previously been shown to modulate acetylcholine release and cholinergic neurotransmission and to reverse cognitive deficits resulting from cholinergic dysfunction in animal models of cognitive impairment and amnesia (van Waarde 2011). Furthermore, abnormalities in the cholinergic system have been reported to be critical for the etiology of mood disorders and could represent an endophenotype of depression (Mineur 2013).

Three of the DEGs identified in the cell type-specific expression analysis, namely Cib2, Npas1, and St8sia2, have been previously linked to HD (Czeredys 2013). Cib2, a calcium and integrin binding family member, is upregulated in the striatum of YAC128 HD mice as well as in a PC12 model of HD (Czeredys 2013). Levels of *Ngas1,* a neuronal PAS domain protein 1 thought to be involved in chromatin remodelling and transcription, are elevated in the PC12 model of HD. *St8sia2,* a ST8 Alpha-N-Acetyl-Neuraminide Alpha-2,8-Sialyltransferase 2 related to ganglioside biosynthesis, has been reported to be decreased in the R6/1 mouse model of HD. The analysis provided herein not only validated these previous findings but also showed that pridopidine treatment (30 mg/kg) reverses the HD-related alterations in the levels of these genes.

In summary, Examples 1-3 demonstrated that pridopidine is a promising therapeutic agent for the treatment of behavioural symptoms in HD patients. Moreover, the experiments provide herein show that such beneficial effects depend on the timing of the treatment, with early treatment leading to greater benefits. The transcriptional analysis provided herein supports a synaptoprotective role for pridopidine and provides novel insights into its mechanisms of action.

### Example 4: Pridopidine is effective in non-HD Rodent Models of Anxiety

Pridopidine is administered periodically (e.g., daily or twice daily) to rodents exhibiting symptoms of anxiety. Examples of rodent models include the HAB rats, selected on the basis of their behavior in the elevated plus maze (EPM); the Syracuse High and Low Avoidance rats; the Maudsley reactive/ nonreactive strains; the Tsukuba High and Low Emotional rats, and the Floripa H and L lines a rat model of anxiety and depression. The Roman Low-Avoidance (RLA) rats, selected on the basis of poor acquisition of a two-way avoidance response in the shuttle box, are considered as a model of high trait anxiety-emotionality. Selective breeding of rats and mice improves the probability of discovering anxiety-related neurobiological correlates, including genetic determinants, and allows the study of gene-environment interactions. (Steimer 2011).

Administering pridopidine is effective in treating anxiety. Administering pridopidine is effective in reducing symptoms of anxiety.

### Example 5: Assessment of Efficacy of Pridopidine In Treating Patients Suffering from Anxiety or Depression.

Pridopidine is administered periodically (e.g., daily or twice daily) to a patient diagnosed with anxiety or depression. The patient is exhibiting symptoms of anxiety or depression. The pridopidine is administered intravenously or orally. Administering pridopidine is effective in treating the patient. Administering pridopidine is also effective in reducing one or more of the symptoms of anxiety or of depression. Administering pridopidine is effective in facilitating rehabilitation of the patient.

Administering pridopidine is effective in facilitating rehabilitation of affective functions of the patient. Administering pridopidine is also effective in facilitating rehabilitation of behavioral function of the patient. Administering pridopidine is also effective in facilitating rehabilitation of emotional function of the patient. Administering pridopidine is also effective in facilitating rehabilitation of psychiatric function of the patient. Administering pridopidine is also effective in facilitating rehabilitation of sensory function of the patient.

### Reference Example 6 : A Phase III, Double-Blind, Placebo-Controlled Study, Evaluating the Safety and Efficacy of Pridopidine 45 mg, versus Placebo for Symptomatic Treatment in Patients with Huntington's Disease

The proposed Phase 3 study is a 78-week, multicenter, randomized, double-blind, placebo controlled, parallel group study to evaluate the efficacy and safety of pridopidine administered at a dose of 45 mg bid in adult patients with early stage HD.

The study consists of a screening period (up to 8 weeks); a 2-week titration period; a 76-week, double-blind, full-dose treatment period; and a follow-up period (consisting of an end of study visit at 3 to 4 weeks after the end of treatment visit).

During the screening period, patients provide informed consent and subsequently undergo assessments to determine eligibility for participation in the study. The stage of HD is established by the UHDRS TFC scale. The PBA-s is assessed.

Eligible patients are invited to return for a baseline visit and baseline assessments. Those patients who remain eligible for study participation will be randomly assigned (1:1 ratio) to 1 of the 2 treatment groups: 45 mg bid pridopidine or placebo bid. For patients assigned to receive pridopidine, the dose is titrated during the first 2 weeks from 45 mg qd to the final dose of 45 mg bid pridopidine.

During titration (days 0 to 14), patients receive 1 scheduled telephone call (TC) during the second week. Patients attend on-site clinic visits at weeks 26, 52, and 78 for safety and efficacy measures and blood sampling for pharmacokinetic assessments. At weeks 3, 6, 12, 39 and 65, safety visits will be conducted either by a visiting nurse at home or at the clinic for safety assessments. Patients will receive 1 scheduled TC approximately 6 to 7 weeks following each at home or on-site clinic visit. During these TCs, patients are asked about the following: adverse events, concomitant medications, alcohol/drug use, tolerability of study drug, use of benzodiazepines and antidepressants, and compliance. The C-SSRS (since last visit version) and abbreviated PBA-s (a subset of PBA-s questions on depressed mood, suicidal ideation, anxiety, irritability, loss of motivation, and obsessive-compulsive behaviors) are assessed.

Patients who complete all scheduled visits have final procedures and assessments performed at the end of treatment visit (week 78). Patients who withdraw from the study before completing the evaluation period will have the week 78 procedures and assessments performed at their final visit, which is considered their early termination visit.

There is an on-site end of study visit approximately 3 to 4 weeks after the last dose of study drug to evaluate efficacy, safety (including a single ECG), pharmacokinetics, rebound, and dependence.

### Problem Behaviors Assessment-Short Form (PBA-s)

Because of the prominence of psychiatric symptoms in HD, it is recommended that the PBA-s form be used in all HD studies with any need for behavioral assessment as a comprehensive screen for the most common psychiatric symptoms in HD. (Craufurd 2001, Kingma 2008)

### REFERENCES

Bech P, et al. "The sensitivity and specificity of the Major Depression Inventory, using the Present State Examination as the index of diagnostic validity.". J Affect Disord. 2001. 66: 159-64.

Bech P, "Rating scales in depression: limitations and pitfalls" Dialogues Clin Neurosci. 2006. 8(2): 207-215.

Bechtel, N. et al., Tapping linked to function and structure in premanifest and symptomatic Huntington disease. Neurology. 2010. 75(24):2150-60.

Benarroch EE. Effects of acetylcholine in the striatum. Recent insights and therapeutic implications. Neurology 2012;79(3):274-281.

Carroll JB et al. Natural history of disease in the YAC128 mouse reveals a discrete signature of pathology in Huntington disease. Neurobiol. Dis. 2011.43(1):257-265.

Connor, KM, et al . Psychometric properties of the Social Phobia Inventory (SPIN) New self-rating scale. The British Journal of Psychiatry, 2000. 176(4), 379-386.

Craufurd D, et al. Behavioral changes in Huntington Disease. Neuropsychiatry Neuropsychol Behav Neurol. 2001. Oct-Dec;14(4):219-26.

Cusin, C, et al. Chapter 2: Rating Scales for Depression, L. Baer, M.A. Blais (eds.), Handbook of Clinical Rating Scales and Assessment in Psychiatry and Mental Health, Current Clinical Psychiatry.

Czeredys M, et al. Expression of genes encoding the calcium signalosome in cellular and transgenic models of Huntington's disease. Front Mol Neurosci 2013. 6:42.

Desplats PA et al. Glycolipid and ganglioside metabolism imbalances in Huntington's disease. Neurobiol. Dis. 2007. 27(3):265-277.

Fenster RJ. Cell-Type Specific Translational Profiling in Huntington's Disease Mouse Models. Student Theses and Dissertations. Paper 148. 2011.

Garcia-Miralles M, et al. L6 Evaluation of pridopidine in the transgenic YAC128 mouse model of Huntington's disease. J Neurol Neurosurg Psychiatry 2016. 87:A92.

Geva , M. et al. Pridopidine activates neuroprotective pathways impaired in Huntington Disease. Human Molecular Genetics, 2016. 25(18):3975-3987

Hamilton M. "The assessment of anxiety states by rating.". Br J Med Psychol. 1959 32 (1): 50-55.

Heimberg, RG, et al. Psychometric properties of the Liebowitz Social Anxiety Scale. Psychological medicine 1999. 29(1), 199.

Huntington Study Group. Unified Huntington's Disease Rating Scale: Reliability and Consistency., Movement Disorders, 1996. 11(2):136-142.

Johnson AC and Paulsen JS. 2014. Huntington's Disease: A Guide for Professionals. D. Lovecky and K. Tarapata eds. Huntington's Disease Society of Americas (HDSA).

Julian, LJ. Measures of anxiety: State-Trait Anxiety Inventory (STAI), Beck Anxiety Inventory (BAI), and Hospital Anxiety and Depression Scale-Anxiety (HADS-A), Arthritis Care & Research November 2011. 63: S11, S467-S472.

Kingma EM, et al. Behavioural problems in Huntington's disease using the Problem Behaviours Assessment. Gen Hosp Psychiatry. 2008. Mar-Apr;30(2):155-6

Kuhn A, et al. Mutant huntingtin's effects on striatal gene expression in mice recapitulate changes observed in human Huntington's disease brain and do not differ with mutant huntingtin length or wild-type huntingtin dosage. Hum Mol Genet 2007. 16(15):1845-1861.

Kuleshov MV et al. Enrichr: a comprehensive gene set enrichment analysis web server 2016 update. Nucleic Acids Research 2016. 44(W1):W90-7.

Leary, MR. A brief version of the Fear of Negative Evaluation Scale. Personality and Social Psychology Bulletin, 1983. 9(3), 371-375.

Mineur, YS. et al. "Cholinergic Signaling in the Hippocampus Regulates Social Stress Resilience and Anxiety- and Depression-like Behavior." PNAS USA 2013.110(9): 3573-3578.

Norman, SB, et al. Development and validation of an overall anxiety severity and impairment scale (OASIS). Depression and anxiety, 2006. 23(4), 245-249.

Pisani A, et al. Re-emergence of striatal cholinergic interneurons in movement disorders. Trends Neurosci 2007. 30(10):545-553.

Ponten H, et al. In vivo pharmacology of the dopaminergic stabilizer pridopidine. Eur J Pharmacol. 2010. 644(1-3):88-95.

Pouladi MA et al. Marked differences in neurochemistry and aggregates despite similar behavioural and neuropathological features of Huntington disease in the full-length BACHD and YAC128 mice. Hum Mol Genet 2012. 21(10):2219-2232.

Shema R et al. Synthetic lethal screening in the mammalian central nervous system identifies Gpx6 as a modulator of Huntington's disease. PNAS 2014. 112(1):268-272.

Slow EJ et al. Selective striatal neuronal loss in a YAC128 mouse model of Huntington disease. Hum Mol Genet 2003. 12(13):1555-1567.

Southwell AL et al. Anti-semaphorin 4D immunotherapy ameliorates neuropathology and some cognitive impairment in the YAC128 mouse model of Huntington disease. Neurobiol. Dis. 2015. 76:46-56.

Spitzer, RL, et al. A brief measure for assessing generalized anxiety disorder: the GAD-7. Archives of internal medicine, 2006. 166(10), 1092.

Steimer T. Animal models of anxiety disorders in rats and mice: some conceptual issues. Dialogues in Clinical Neuroscience. 2011.13(4):495-506.

Strik J, et al. Sensitivity and specificity of observer and self-report questionnaires in major and minor depression following myocardial infarction. Psychosomatics. 2001. 42:423-428.

Teo RTY et al. Structural and molecular myelination deficits occur prior to neuronal loss in the YAC128 and BACHD models of Huntington disease. Hum Mol Genet 2016. 25(13):2621-2632.

van Roon-Mom WMC et al. Mutant huntingtin activates Nrf2-responsive genes and impairs dopamine synthesis in a PC12 model of Huntington's disease. BMC Mol Biol 2008. 9:84.

van Waarde A et al. The cholinergic system, sigma-1 receptors and cognition. Behav Brain Res 2011. 221(2):543-554.

Xie, Y, et al. BDNF Overexpression in the Forebrain Rescues Huntington's Disease Phenotypes in YAC128 Mice. J Neurosci. 2010. 30(44): 14708-14718.

Zigmond, AS, and Snaith, RP.. The hospital anxiety and depression scale. Acta psychiatrica scandinavica, 1983. 67(6), 361-370.

CSID:25948790, www.chemspider.com/Chemical-Structure.25948790.html (accessed 23:27, Jul 15, 2016).

CSID:7971505, www.chemspider.com/Chemical-Structure.7971505.html (accessed 23:33, Jul 15, 2016).

U.S. Patent No. 6,903,120, issued June 7, 2005 (Sonesson et al.).

U.S. Patent No. 7,923,459, issued April 12, 2011 (Gauthier et al.).

US Patent Publication No. US2016/243098 published Aug. 25, 2016 (Geva et al).

## Claims

1. A pharmaceutical composition comprising pridopidine for use in reducing anxiety and/or depression in a subject in need thereof, wherein the anxiety or depression is not caused by Huntington's Disease.

2. The pharmaceutical composition for use according to claim 1, wherein the anxiety is reduced by at least one scale unit of measure in the State-Trait Anxiety Inventory (STAI), the Fear Survey Schedule, Beck Anxiety Inventory (BAI), Brief Fear of Negative Evaluation Scale - BFNE, Clinician Administered PTSD Scale (CAPS), Daily Assessment of Symptoms - Anxiety, Generalized Anxiety Disorder 7 (GAD-7), Hamilton Anxiety Scale (HAM-A), Hospital Anxiety and Depression Scale (HADS-A), Leibowitz Social Anxiety Scale (LSAS), Overall Anxiety Severity and Impairment Scale (OASIS), Panic and Agoraphobia Scale (PAS), Panic Disorder Severity Scale (PDSS), PTSD Symptom Scale - Self-Report Version, Social Phobia Inventory (SPIN), Trauma Screening Questionnaire, Yale-Brown Obsessive Compulsive Scale (Y-BOCS), or the Zung Self-Rating Anxiety Scale.

3. The pharmaceutical composition for use according to claim 1 or 2, wherein the depression is reduced by at least one scale unit of measure in the Hamilton Rating Scale for Depression (HAM-D), Beck Depression Inventory (BDI), Beck Hopelessness Scale, Centre for Epidemiological Studies - Depression Scale (CES-D), Patient Health Questionnaire, Center for Epidemiological Studies Depression Scale for Children (CES-DC), Clinically Useful Depression Outcome Scale, Diagnostic Inventory for Depression, Edinburgh Postnatal Depression Scale (EPDS), Inventory of Depressive Symptomatology, Geriatric Depression Scale (GDS), Hospital Anxiety and Depression Scale, Kutcher Adolescent Depression Scale (KADS), Major Depression Inventory (MDI), Montgomery-Åsberg Depression Rating Scale (MADRS), Mood and Feelings Questionnaire (MFQ), Zung Self-Rating Depression Scale, or the Cornell Scale for Depression in Dementia (CSDD).

4. The pharmaceutical composition for use according to any of claims 1-3, wherein the subject is afflicted with an anxiety disorder which is generalized anxiety disorder (GAD), panic disorder, a phobic disorder, social phobia, agoraphobia, or a trauma- or stressor-related disorder.

5. The pharmaceutical composition for use according to any of claims 1-3, wherein the subject is afflicted with a depressive disorder which is major depressive disorder, persistent depressive disorder, premenstrual dysphoric disorder, other depressive disorder, depressive disorder due to another medical condition, substance/medication-induced depressive disorder, perinatal depression, peripartum-onset depression, seasonal affective disorder, or psychotic depression.

6. The pharmaceutical composition for use according to claim 1, wherein the subject has been diagnosed with anxiety only.

7. The pharmaceutical composition for use according to claim 1, wherein the subject has been diagnosed with depression only.

8. The pharmaceutical composition for use according to any of the preceding claims, wherein between 22.5 - 315 mg pridopidine is administered to the subject per day.

9. The pharmaceutical composition for use according to any of the preceding claims, wherein the unit dose is administered once daily, more than once daily, or twice per day.

10. The pharmaceutical composition for use according to any of the preceding claims, wherein the composition is administered orally.

11. The pharmaceutical composition for use according to any of the preceding claims, wherein the pridopidine is in the form of pridopidine hydrochloride, hydrobromide, nitrate, perchlorate, phosphate, the sulphate, formate, acetate, aconate, ascorbate, benzenesulphonate, benzoate, cinnamate, citrate, embonate, enantate, fumarate, glutamate, glycolate, lactate, maleate, malonate, mandelate, methanesulphonate, naphthalene-2-sulphonate, phthalate, salicylate, sorbate, stearate, the succinate, tartrate, or toluene-p-sulphonate salt thereof.

## Patentansprüche

1. Pharmazeutische Zusammensetzung, die Pridopidin enthält, zur Verwendung bei der Linderung von Angst und/oder Depressionen bei einer Person, die dies benötigt, wobei die Angst oder Depression nicht durch die Huntington-Krankheit verursacht wird.

2. Pharmazeutische Zusammensetzung zur Verwendung nach Anspruch 1, wobei die Angst um mindestens eine Maßeinheit nach dem State-Trait Anxiety Inventory (STAI), dem Fear Survey Schedule, dem Beck-Angst-Inventar (BAI), der Skala Brief Fear of Negative Evaluation (BFNE), der Skala Clinician Administered PTSD (CAPS), der Daily Assessment of Symptoms - Anxiety, Generalized Anxiety Disorder 7 (GAD-7), der Hamilton-Angst-Skala (HAM-A), der Hospital Anxiety and Depression Scale (HADS-A), der Liebowitz Social Anxiety Scale (LSAS), der Overall Anxiety Severity and Impairment Scale (OASIS), der Panik und Agoraphobie-Skala (PAS), der Panic Disorder Severity Scale (PDSS), PTBS-Symptomskala - Selbstberichtversion, dem Soziale-Phobie-Inventar (SPIN), dem Trauma-Screening-Fragebogen, der Yale-Brown-Skala für Zwangsstörungen (Y-BOCS) oder der Zung-Selbstbeurteilungsskala für Angst vermindert wird.

3. Pharmazeutische Zusammensetzung zur Verwendung nach Anspruch 1 oder 2, wobei die Depressionen um mindestens eine Maßeinheit nach der Hamilton-Skala (HAM-D), dem Beck-Depressions-Inventar (BDI), der Beck-Hoffnungslosigkeits-Skala, der Skala für Depression des Zentrums für Epidemiologische Studien (CES-D), dem Patientengesundheitsfragebogen, der Skala für Depression des Zentrums für Epidemiologische Studien für Kinder (CES-DC), der Clinically Useful Depression Outcome Scale, dem diagnostischen Inventar für Depressionen, der Edinburgh-Postnatal-Depressionsskala Depression Scale (EPDS), dem Inventar depressiver Symptome, der Geriatrischen Depressions-Skala (GDS), der Hospital Anxiety and Depression Scale, der Kutcher Adolescent Depression Scale (KADS), dem Major Depression Inventory (MDI), der Montgomery-Asberg Depression Rating Scale (MADRS), dem Mood and Feelings Questionnaire (MFQ), der Zung Self-Rating Depression Scale oder der Cornell Scale for Depression in Dementia (CSDD) vermindert werden.

4. Pharmazeutische Zusammensetzung zur Verwendung nach einem der Ansprüche 1-3, wobei die Person an einer Angststörung leidet, bei der es sich um eine generalisierte Angststörung (GAD), eine Panikstörung, eine phobische Störung, eine soziale Phobie, eine Agoraphobie oder eine trauma- oder stressbedingte Störung handelt.

5. Pharmazeutische Zusammensetzung zur Verwendung nach einem der Ansprüche 1-3, wobei die Person an einer depressiven Störung leidet, bei der es sich um eine schwere depressive Störung, eine persistierende depressive Störung, eine prämenstruelle dysphorische Störung, eine andere depressive Störung, eine depressive Störung aufgrund einer anderen Erkrankung, eine durch eine Substanz/ein Medikament induzierte depressive Störung, eine perinatale Depression, eine peripartale Depression, eine saisonal-affektive Störung oder eine psychotische Depression handelt.

6. Pharmazeutische Zusammensetzung zur Verwendung nach Anspruch 1, wobei bei der Person nur Angst diagnostiziert wurde.

7. Pharmazeutische Zusammensetzung zur Verwendung nach Anspruch 1, wobei bei der Person nur Depressionen diagnostiziert wurden.

8. Pharmazeutische Zusammensetzung zur Verwendung nach einem der vorhergehenden Ansprüche, wobei der Person pro Tag zwischen 22,5 und 315 mg Pridopidin verabreicht werden.

9. Pharmazeutische Zusammensetzung zur Verwendung nach einem der vorhergehenden Ansprüche, wobei die Einheitsdosis einmal täglich, mehr als einmal täglich oder zweimal täglich verabreicht wird.

10. Pharmazeutische Zusammensetzung zur Verwendung nach einem der vorhergehenden Ansprüche, wobei die Zusammensetzung oral verabreicht wird.

11. Pharmazeutische Zusammensetzung zur Verwendung nach einem der vorhergehenden Ansprüche, wobei das Pridopidin in Form von Pridopidinhydrochlorid, -hydrobromid, -nitrat, -perchlorat, -phosphat, -sulfat, -formiat, -acetat, -aconat, -ascorbat, -benzolsulfonat, -benzoat, -cinnamat, -citrat, -embonat, -enantat, -fumarat, -glutamat, -glykolat, -lactat , Maleat, Malonat, Mandelsäureester, Methansulfonat, Naphthalin-2-sulfonat, Phthalat, Salicylat, Sorbat, Stearat, Succinat, Tartrat oder Toluol-p-sulfonat-Salz davon vorliegt.

## Revendications

1. Composition pharmaceutique comprenant de la pridopidine pour une utilisation dans la réduction de l'anxiété et/ou de la dépression chez un sujet en ayant besoin, l'anxiété ou la dépression n'étant pas provoquée par la maladie de Huntington.

2. Composition pharmaceutique pour une utilisation selon la revendication 1, l'anxiété étant réduite d'au moins un incrément de mesure dans l'inventaire d'anxiété état-trait (STAI), le programme d'enquête sur la peur, l'inventaire d'anxiété de Beck (BAI), version brève de l'échelle d'évaluation de peur de l'évaluation négative - BFNE, l'échelle du SSPT administrée par un clinicien (CAPS), l'évaluation quotidienne des symptômes - anxiété, le trouble d'anxiété généralisée 7 (GAD-7), l'échelle d'anxiété de Hamilton (HAM-A), l'échelle d'anxiété et de dépression hospitalière (HADS-A), l'échelle d'anxiété sociale de Leibowitz (LSAS), l'échelle de gravité et d'altération de l'anxiété globale (OASIS), l'échelle de panique et d'agoraphobie (PAS), l'échelle de gravité du trouble panique (PDSS), l'échelle des symptômes du SSPT - version autodéclarée, l'inventaire de la phobie sociale (SPIN), le questionnaire de dépistage des traumatismes, l'échelle des troubles obsessionnels compulsifs de Yale-Brown (Y-BOCS) ou l'échelle d'autodétermination de l'anxiété de Zung.

3. Composition pharmaceutique pour une utilisation selon la revendication 1 ou 2, la dépression étant réduite d'au moins un incrément de mesure dans l'échelle d'évaluation de Hamilton pour la dépression (HAM-D), l'inventaire de dépression de Beck (BDI), l'échelle de désespoir de Beck, l'échelle de dépression du Centre d'études épidémiologiques (CES-D), le questionnaire de santé du patient, l'échelle de dépression pour les enfants du Centre d'études épidémiologiques (CES-DC), l'échelle de résultats de dépression cliniquement utiles, l'inventaire diagnostique de la dépression, l'échelle de dépression postnatale d'Édimbourg (EPDS), l'inventaire de la symptomatologie dépressive, l'échelle de dépression gériatrique (GDS), l'échelle d'anxiété et de dépression hospitalière, l'échelle de dépression de Kutcher pour les adolescents (KADS), l'inventaire de dépression majeure (MDI), l'échelle d'évaluation de la dépression de Montgomery-Asberg (MADRS), le questionnaire sur l'humeur et les sentiments (MFQ), l'échelle d'autodétermination de la dépression de Zung ou l'échelle de Cornell pour la dépression dans la démence (CSDD).

4. Composition pharmaceutique pour une utilisation selon l'une quelconque des revendications 1 à 3, le sujet étant atteint d'un trouble anxieux qui est un trouble anxieux généralisé (TAG), un trouble panique, un trouble phobique, une phobie sociale, une agoraphobie ou des troubles liés à un traumatisme ou à un facteur de stress.

5. Composition pharmaceutique pour une utilisation selon l'une quelconque des revendications 1 à 3, le sujet étant atteint d'un trouble dépressif qui est un trouble dépressif majeur, un trouble dépressif persistant, un trouble dysphorique prémenstruel, un autre trouble dépressif, un trouble dépressif dû à un autre état médical, un trouble dépressif induit par une substance/un médicament, une dépression périnatale, une dépression d'apparition péripartum, un trouble affectif saisonnier ou une dépression psychotique.

6. Composition pharmaceutique pour une utilisation selon la revendication 1, le sujet ayant été diagnostiqué comme souffrant uniquement d'anxiété.

7. Composition pharmaceutique pour une utilisation selon la revendication 1, le sujet ayant été diagnostiqué comme souffrant uniquement de dépression.

8. Composition pharmaceutique pour une utilisation selon l'une quelconque des revendications précédentes, entre 22,5 et 315 mg de pridopidine étant administrés au sujet par jour.

9. Composition pharmaceutique pour une utilisation selon l'une quelconque des revendications précédentes, la dose unitaire étant administrée une fois par jour, plus d'une fois par jour ou deux fois par jour.

10. Composition pharmaceutique pour une utilisation selon l'une quelconque des revendications précédentes, la composition étant administrée par voie orale.

11. Composition pharmaceutique pour une utilisation selon l'une quelconque des revendications précédentes, dans laquelle la pridopidine se présente sous la forme d'un sel chlorhydrate, bromhydrate, nitrate, perchlorate, phosphate, sulphate, formiate, acétate, aconate, ascorbate, benzènesulphonate, benzoate, cinnamate, citrate, embonate, enantate, fumarate, glutamate, glycolate, lactate, maléate, malonate, mandélate, méthanesulphonate, naphthalène-2-sulphonate, phthalate, salicylate, sorbate, stéarate, succinate, tartrate ou toluène-p-sulphonate, de pridopidine.
